# EUROPÄISCHE PATENTSCHRIFT

(11) **EP 1 383 763 B1**
(45) Veröffentlichungstag und Bekanntmachung des Hinweises auf die Patenterteilung: **12.10.2005**
(21) Anmeldenummer: 02727539.5
(22) Anmeldetag: 08.04.2002
(51) Int. Cl.: C07D 405/12, C07D 311/58

(54) **VERFAHREN ZUR HERSTELLUNG VON 2-(-5-(4-FLUORPHENYL)-3-PYRIDYLMETHYLAMINOMETHYL)-CHROMAN**
METHOD FOR PRODUCING 2-(-5-(4-FLUOROPHENYL)-3-PYRIDYLMETHYLAMINOMETHYL)-CHROMANE
PROCEDE DE FABRICATION DE 2-(-5-(4-FLUOROPHENYL)-3-PYRIDYLMETHYLAMINOMETHYL)-CHROMANE

(30) Priorität: 26.04.2001 DE 10120619
(43) Veröffentlichungstag der Anmeldung: 28.01.2004
(73) Patentinhaber: Merck Patent GmbH, 64293 Darmstadt (DE)
(72) Erfinder: BOKEL, Heinz-Hermann, D-64287 Darmstadt (DE); NEUNFELD, Steffen, 64409 Messel (DE); GANTZERT, Ludwig, 64319 Pfungstadt (DE); KNIERIEME, Ralf, 64846 Gross-Zimmern (DE); SIMON, Elke, 64846 Gross-Zimmern (DE); DEVANT, Ralf, 64291 Darmstadt (DE); HELM, Udo, 64720 Michelstadt (DE); REUBOLD, Helmut, 64732 Bad König (DE)
(86) Internationale Anmeldenummer: PCT/EP2002/003857
(87) Internationale Veröffentlichungsnummer: WO 2002/088117

(56) Entgegenhaltungen:
- DE-A- 10 005 150
- FR-A- 2 701 479
- US-A- 5 318 988
- US-A- 5 767 132
- J. FALBE (HRSG.): "Römpp-Lexikon Chemie" 1999 , GEORG THIEME VERLAG , STUTTGART XP002212624 Abschnitt Racemattrennung Seite 3693

## Beschreibung

Die Erfindung betrifft ein Verfahren zur Herstellung von 2-[5-(4-Fluorphenyl)-3-pyridylmethylaminomethyl]-chroman der Formel I, 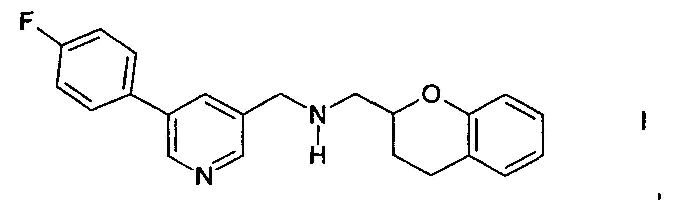 einschließlich der enantiomerenreinen Verbindungen der Formel 1, und dessen Salzen, dadurch gekennzeichnet, dass 5-(4-Fluorphenyl)-pyridin-3-carbaldehyd direkt mit 2-Aminomethyl-chroman oder dessen Salzen unter reduzierenden Bedingungen zu der Verbindung der Formel I umgesetzt wird und gegebenenfalls die erhaltene Verbindung der Formel I durch Behandeln mit einer Säure in eines ihrer Salze umgewandelt wird.

Die Verbindung 2-[5-(4-Fluorphenyl)-3-pyridylmethylaminomethyl]-chroman ist sowohl als Racemat als auch als enantiomerenreine Verbindungen aus US 5,767,132 bekannt. In US 5,767,132 wird ebenfalls die Herstellung der physiologisch unbedenklichen Salze (Spalte 9, Zeilen 6 bis 32) und ein Herstellungsprozess (Beispiele 5 und 19) beschrieben. 2-[5-(4-Fluorphenyl)-3-pyridylmethylaminomethyl]-chroman der Formel I und dessen Salze sind selektive Dopamin D₂ Rezeptor Antagonisten und 5-HT_{1A} Rezeptor Agonisten. Sie sind daher für die Verwendung zur Herstellung eines Arzneimittels zur Prophylaxe und/oder Behandlung von Folgeerkrankungen nach cerebralem Infarkt (apoplexia cerebri), beispielsweise Schlaganfall und cerebraler Ischämien, zur Prophylaxe und Kontrolle von cerebralen Krankheiten, beispielsweise Migräne, der Behandlung von Angst-, Spannungs- und Depressionszuständen, zentralnervös bedingten Sexualdysfunktionen, Schlaf- und Nahrungsaufnahmestörungen oder zur Behandlung von Psychosen, beispielsweise Schizophrenien, schizoaffektiven Psychosen oder Zyklothymien geeignet.
Unter Angstzuständen sind auch die Krankheitsbilder Panikstörung mit und ohne Agoraphobie, Zwangsstörungen oder zwanghafte Persönlichkeitsstörung, Spezifische Angsstörung, Soziale Angststörung, akute Belastungsstörung, posttraumatische Belasungsstörung, generalisierte Angststörung, substanzinduzierte Angststörung und auch eine Angststörung aufgrund eines medizinischen Krankheitsfaktors zu verstehen.

2-[5-(4-Fluorphenyl)-3-pyridylmethylaminomethyl]-chroman der Formel I und dessen Salze sind weiterhin zur Beseitigung kognitiver Defizite, zur Verbesserung von Lern- und Gedächtnisleistungen und zur Behandlung der Alzheimer'schen Krankheit geeignet. Sie können ebenfalls zur Behandlung von Nebenwirkungen bei der Hypertonie-Behandlung, in der Endokrinologie und Gynäkologie, beispielsweise zur Behandlung der Akromegalia, Hypogonadismus, sekundärer Amenorrhoe, prämenstruellem Syndrom oder unerwünschter puerperalen Laktation eingesetzt werden.

Die Verbindungen können daher als Arzneimittetwirkstoffe in der Human- und Veterinärmedizin verwendet werden. Ferner können sie als Zwischenprodukte zur Herstellung weiterer Arzneimittelwirkstoffe Verwendung finden.

Da 2-[5-(4-Fluorphenyl)-3-pyridylmethylaminomethyl]-chroman, insbesondere (R)-(-)-2-[5-(4-Fluorphenyl)-3-pyridylmethylaminomethyl]-chroman aber auch (S)-(+)-2-[5-(4-Fluorphenyl)-3-pyridylmethylaminomethyl] chroman, sowie dessen Salze als Arzneimittelwirkstoffe sehr vielversprechend sind, ist die Herstellung von größtem Interesse.

Aufgabe der vorliegenden Erfindung war daher, eine neue und effektive Synthesevariante für die zuvor beschriebenen Verbindungen zu finden.

Bei der bisher bekannten Synthese von 2-[5-(4-Fluorphenyl)-3-pyridylmethylaminomethyl]-chroman als Racemat sowie als enantiomerenreine Verbindungen, beschrieben in US 5,767,132, Beispiele 5 und 19, wird 2-Aminomethyl-chroman, sowohl als Racemat als auch als enantiomerenreine Verbindung oder ein entsprechendes Salz dieser Verbindungen mit 3-(Chlormethyl)-5-(4-fluorphenyl)-pyridin umgesetzt. Das Edukt 3-(Chlormethyl)-5-(4-flurophenyl)-pyridin ist jedoch hautreizend und kann Allergien erzeugen. Weiterhin besitzen "Halogen-aminoverbindungen", wie z.B. 3-(Chlormethyl)-5-(4-flurophenyl)-pyridin, eine Neigung zu exothermer Zersetzung, da gleichzeitig alkylierende und alkylierbare funktionelle Gruppen in einem einzigen Molekül vorhanden sind (Lit: Chem.-Ing.-Tech. 1979, 51, 928-933).

Gegenstand der Erfindung ist daher ein Verfahren zur Herstellung von 2-[5-(4-Fluorphenyl)-3-pyridylmethylaminomethyl]-chroman der Formel I, 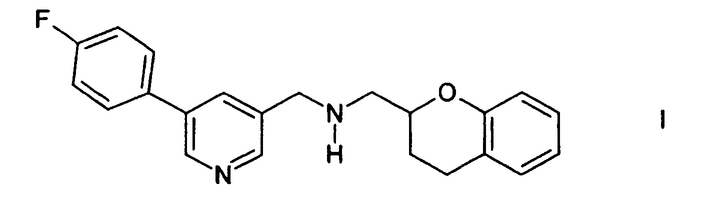 und dessen Salzen, dadurch gekennzeichnet, dass
(1) 5-(4-Fluorphenyl)-pyridin-3-carbaldehyd der Formel II 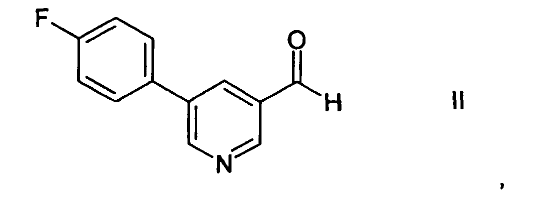 direkt mit 2-Aminomethyl-chroman oder dessen Salzen unter reduzierenden Bedingungen zu der Verbindung der Formel I umgesetzt wird und
(2) die erhaltene Verbindung der Formel I durch Behandeln mit einer Säure in eines ihrer Salze umgewandelt wird.

Der Vorteil des neuen Verfahrens gegenüber dem aus US 5,767,132 bekannten Verfahren liegt in der Verringerung von Nebenprodukt durch Unterdrückung der Doppel-Alkylierung. Der Wirkstoff erhält nach Umstellung auf das Verfahren kein Nebenprodukt mit zwei Arylpyridinresten, was die Aufreinigung des Wirkstoffs erleichtert.

Im Vergleich zu aus der Standardliteratur bekannten Herstellungsvarianten von sekundären Aminen durch Reaktion von Aldehyden mit primären Aminen (Lit.: Houben-Weyl, Methoden der organischen Chemie, Band 11/1), ist es bei dem erfindungsgemäßen Verfahren nicht erforderlich, die Wasserabspaltung zum Aldimin als Zwischenprodukt zu forcieren. Stattdessen kann der Aldehyd, erfindungsgemäß der 5-(4-Fluor-phenyl)-pyridin-3-carbaldehyd, direkt mit 2-Aminomethyl-chroman unter reduzierenden Bedingungen zusammengegeben werden.

Der Vorteil gegenüber den unter Standardbedingungen erfolgenden reduktiven Aminierungen besteht folglich darin, dass eine geringere Zahl von Reagenzien und eine geringere thermische Belastung erforderlich ist. Dies führt ebenfalls zu weniger Verunreinigungen und Nebenreaktionen.

Insbesondere kann auf ein mehrstündiges Erhitzen in einem azeotoropbildenden Lösungsmittel unter Zusatz eines Katalysators, d.h. einer Säure, beispielsweise von p-Toluolsulfonsäure, verzichtet werden. Typische Nebenreaktionen thermisch belasteter Aldehyde sind beispielsweise Disproportionierung zu Alkohol und Säure oder Oligomerisierung unter Wasserabspaltung, beispielsweise zum substituierten Trioxan.

Zusätzlich wurde gefunden, dass die reaktive Form des 2-Aminomethyl-chromans (freie Base) nicht separat hergestellt werden muss, sondern in situ direkt aus einer lagerstabilen Salzform des Amins hergestellt werden kann. Durch den Verzicht auf die Isolierung der freien Base entfällt mindestens eine flüssig/flüssig Verteilung. Dadurch wird der Lösemittelverbrauch zusätzlich eingeschränkt.

Gegenstand der Erfindung ist daher das Verfahren, wie zuvor beschrieben, dadurch gekennzeichnet, dass die reaktive Form des 2-Aminomethyl-chromans in situ aus einem Salz des 2-Aminomethyl-chromans hergestellt wird.
Besonders geeignete Salze des 2-Aminomethyl-chromans sind 2-Aminomethyl-chroman Maleat, 2-Aminomethyl-chroman Hydrochlorid, 2-Aminomethyl-carbonat. Besonders vorteilhaft ist erfindungsgemäß die Verwendung von 2-Aminomethyl-chroman Hydrochlorid.

2-[5-(4-Fluorphenyl)-3-pyridylmethylaminomethyl]-chroman der Formel I enthält ein chirales Zentrum in 2-Position des Chromangerüsts und kann daher in racemischer oder in optisch-aktiver Form vorliegen. Die Formel I umfaßt sowohl das Racemat als auch die enantiomerenreinen Verbindungen (R)-(-)-2-[5-(4-Fluorphenyl)-3-pyridylmethylaminomethyl]-chroman und (S)-(+)-2-[5-(4-Fluorphenyl)-3-pyridylmethylaminomethyl]-chroman.
Racemate, wie auch das Racemat von 2-[5-(4-Fluorphenyl)-3-pyridylmethylaminomethyl]-chroman, können nach an sich bekannten Methoden mechanisch oder chemisch in die Isomeren getrennt werden. Vorzugsweise werden aus dem racemischen Gemisch durch Umsetzung mit einem optisch aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder Aminosäuren oder die verschiedenen optisch aktiven Camphersulfonsäuren wie β-Camphersulfonsäure.
Vorteilhaft ist auch eine chromatographische Enantiomerentrennung mit Hilfe einer mit einem optisch aktiven Trennmittel (z.B. Dinitrobenzoylphenylglycin) gefüllten Säule; als Laufmittel eignet sich z.B. ein Gemisch Hexanl/Isopropanol/Acetonitril, z.B. im Volumenverhältnis 82:15:3 oder wie beispielsweise in WO 97/47617 beschrieben.
Eine Diastereomerentrennung kann auch durch Standardreinigungsprozesse, wie z.B. fraktionierte Kristallisation erfolgen (Lit.: A. Collet, S. H. Wilen, Enantiomers, Racemates and Resolutions, Wiley (New York) 1981).

Natürlich ist es auch möglich, optisch aktive Verbindungen der Formel I nach den oben beschriebenen Verfahren zu erhalten, indem man 2-Aminomethyl-chroman verwendet, das bereits optisch aktiv ist [(R)-2-Aminomethyl-chroman oder (S)-2-Aminomethyl-chroman].

Racemisches 2-Aminomethyl-chroman ist kommerziell erhältlich oder kann nach bekannten Syntheseverfahren hergestellt werden.
(R)- oder (S)-2-Aminomethyl-chroman kann nach bekannten Syntheseverfahren hergestellt werden.
Die Herstellung kann beispielsweise ausgehend von der kommerziell erhältlichen Chroman-2-carbonsäure durch folgende Umsetzungen erfolgen:
(1) Reaktion mit Thionylchlorid zum Carbonsäurechlorid,
(2) Umsetzung mit einem chiralen Amin zu einem Diastereomerengemisch des Carbonsäureamids,
(3) Diastereomerentrennung nach herkömmlichen Methoden wie zuvor beschrieben,
(4) Reduktion des diastereomeren Carbonsäureamids zu dem korrespondierenden N-substituierten 2-Aminomethyl-chroman und
(5) Dealkylierung, beispielsweise durch katalytische Hydrierung zum enantiomerenreinen 2-Aminomethylchroman. Wahlweise, je nach Konfiguration des chiralen Amins erhält man das (R)- oder das (S)-Enantiomere.

Gegenstand der Erfindung ist ebenfalls ein Verfahren zur Herstellung von (R)-(-)-2-[5-(4-Fluorphenyl)-3-pyridylmethylaminomethyl]-chroman der Formel la, 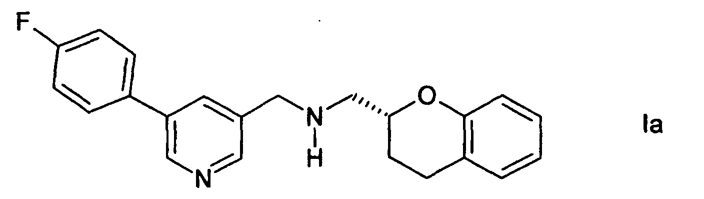 und dessen Salzen, dadurch gekennzeichnet, dass
(1) 5-(4-Fluorphenyl)-pyridin-3-carbaldehyd der Formel II 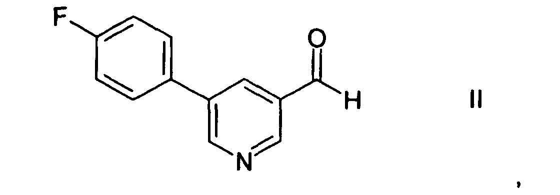 direkt mit (R)-2-Aminomethyl-chroman oder dessen Salzen unter reduzierenden Bedingungen zu der Verbindung der Formel la umgesetzt wird und
(2) die erhaltene Verbindung der Formel la durch Behandeln mit einer Säure in eines ihrer Salze umgewandelt wird. In analoger Weise wird unter Verwendung von (S)-2-Aminomethylchroman das (S)-Enantiomer der Verbindung I erhalten.

Alternativ kann die Herstellung des (R)-2-Aminomethyl-chromans auch durch Umsetzung von racemischen 2-Aminomethyl-chroman mit (S)-N-Tosylprolin und sich anschließender Racematspaltung durch Kristallisation erfolgen. Die Löslichkeiten der beiden diastereomeren Salze des racemischen Amins mit enantiomerenreinen N-Tosyl-(S)-Prolin sind so unterschiedlich, dass das reine (R)/(S)-Diastereomere der Formel III durch normale Kristallisation erhalten werden kann. Es entsteht folglich (R)-2-Aminomethyl-chroman N-(Tosylsulfonyl)-(S)-prolinat der Formel III 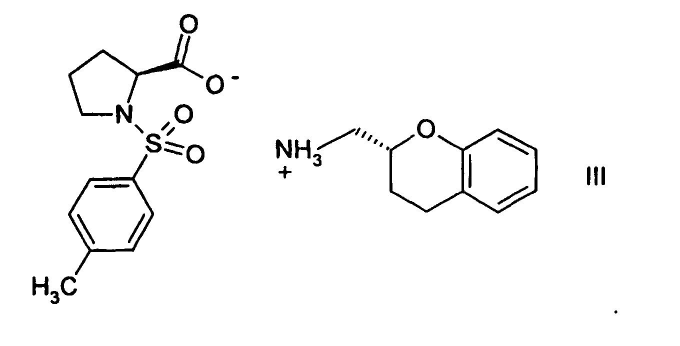 Enantiomerenreines (R)-2-Aminomethylchroman wird anschliessend aus der Verbindung der Formel III mittels basischer Extraktion freigesetzt. In analoger Weise erhält man unter Verwendung von (R)-N-Tosylprolin das (S)-2-Aminomethyl-chroman.

5-(4-Fluorphenyl)-pyridin-3-carbaldehyd kann nach bekannten Syntheseverfahren hergestellt werden. Die Herstellung kann beispielsweise ausgehend von der kommerziell erhältlichen 5-(4-Fluorphenyl)nicotinsäure durch folgende Umsetzungen erfolgen:
(1) Reduktion zum 5-(4-Fluorphenyl)-3-hydroxymethylpyridin, beispielsweise in Gegenwart von Borhydriden, und nachfolgende
(2) Oxidation des Alkohols, beispielsweise mit Braunstein (MnO₂) zum gewünschten Aldehyd.

Eine weitere Alternative ist die Herstellung des Aldehyds durch Reduktion des 3-Cyano-5-(4-fluorphenyl)-pyridins. z.B. durch Hydrierung oder mit Hydriden wie Diisobutylaluminiumhydrid oder Lithium-tri-tert.butoxyaluminiumhydrid.

Die Umsetzung des 5-(4-Fluorphenyl)-pyridin-3-carbaldehyds mit 2-Aminomethyl-chroman Hydrochlorid, insbesondere (R)-2-Aminomethylhydrochlorid, findet unter reduzierenden Reaktionsbedingungen statt, beispielsweise in Gegenwart von Borhydriden oder Hydrierkatalysatoren.

Geeignete Borhydride sind Lithiumborhydrid, Natriumborhydrid, Natriumcyanoborhydrid, Kaliumborhydrid oder Borhydrid auf polymeren Trägermaterialien, beipielsweise Amberlyst A-26 BH₄⁻-Form. Besonders bevorzugt wird Natriumborhydrid eingesetzt, das zuvor mit Methanol zu Natrium-trimethoxyborhydrid umgesetzt wird.

Für die Umsetzung in Gegenwart von Borhydriden ist jedes Lösungsmittel geeignet, sofern es nicht mit den Edukten interferiert. Besonders geeignet sind protische Lösungsmittel, beispielsweise Alkohole wie Ethanol oder Methanol oder Mischungen davon.
Geeignete Reaktionstemperaturen liegen zwischen 0° und 70°, bevorzugt zwischen 10 und 50°, besonders bevorzugt zwischen 20 und 35°C.

Gegenstand der Erfindung ist auch ein Verfahren, wie zuvor beschrieben, dadurch gekennzeichnet, dass die Umsetzung in Gegenwart eines Borhydrids stattfindet.

Die Umsetzung des 5-(4-Fluorphenyl)-pyridin-3-carbaldehyds mit 2-Aminomethyl-chroman Hydrochlorid, insbesondere (R)-2-Aminomethylhycrochlorid, kann ebenfalls in Anwesenheit von Wasserstoffgas und Hydrierkatalysatoren erfolgen.

Geeignete Hydrierkatalysatoren sind beispielsweise Metalle der achten Nebengruppe, beispielsweise Raney-Nickel, Palladium oder Platin. Palladium- oder Platin-Katalysatoren können auf einem Trägermaterial vorliegen, beispielsweise auf Aktivkohle, Kalziumcarbonat, Bariumsulfat oder Strontiumcarbonat, in der Form ihrer Oxide, beispielsweise Platinoxid oder in fein verteilter Form.

Bevorzugt wird bei einem Druck von 1 bis 200 bar und bei Temperaturen zwischen -80° und +150°C gearbeitet, besonders bevorzugt bei Raumtemperatur und Normaldruck.

Als Lösungsmittel sind beispielsweise Alkohole, wie Methanol, Ethanol oder Isopropanol, Carbonsäuren wie Essigsäure, Ester wie Ethylacetat oder Ether wie Tetrahydrofuran (THF) oder Dioxan geeignet. Es ist ebenfalls möglich, Lösungsmittelgemische der oben genannten Lösungsmittel einzusetzen, gegebenenfalls auch Lösungsmittelgemische enthaltend Wasser.

Gegenstand der Erfindung ist daher ein Verfahren wie zuvor beschrieben, dadurch gekennzeichnet, dass die Umsetzung in Gegenwart von Wasserstoff und einem Hydrierkatalysator stattfindet.

Die erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure; Benzolsulfonsäure, p-Toluolsulfonsäure, Naphthalin-mono- und -disulfonsäuren, Laurylschwefelsäure.

In einer bevorzugten Ausführungsform erfolgt die Salzbildung in Ethanol durch Fällung mit Salzsäure (37%). Es entsteht 2-[5-(4-Fluorphenyl)-3-pyridylmethylaminomethyl]-chroman Dihydrochtorid-Hemihydrat oder (R)-(-)-2-[5-(4-Fluorphenyl)-3-pyridylmethylaminomethyl]-chroman Dihydrochlorid-Hemihydrat.

Gegenstand der Erfindung ist das Verfahren, wie zuvor beschrieben, dadurch gekennzeichnet, dass (R)-(-)-2-[5-(4-Fluorphenyl)-3-pyridylmethylaminomethyl]-chroman Dihydrochlorid hergestellt wird.

In einer ebenfalls bevorzugten Ausführungsform erfolgt die Salzbildung bei -5°C durch Zugabe der stöchiometrischen Menge 37%-iger wässriger Salzsäure zu einer 7%-igen Lösung der Base in Ethanol. Man erhält (R)-(-)-2 -[5-(4-Fluorphenyl)-3-pyridylmethylaminomethyl]-chroman Hydrochlorid.

Gegenstand der Erfindung ist insbesondere das Verfahren, wie zuvor beschrieben, dadurch gekennzeichnet, dass (R)-(-)-2-[5-(4-Fluorphenyl)-3-pyridylmethylaminomethyl]-chroman Hydrochlorid hergestellt wird.

Auch ohne weitere Ausführungen wird davon ausgegangen, dass ein Fachmann die obige Beschreibung in weitestem Umfang nutzen kann. Die bevorzugten Ausführungsforrnen sind deswegen lediglich als beschreibende, keineswegs als in irgendeiner Weise limitierende Offenbarung aufzufassen.

Alle vorstehenden und nachfolgenden Temperaturangaben werden in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

### Beispiel 1:

20,3 g (R)-2-Aminomethylchroman Hydrochlorid werden in 150 ml Ethanol vorgelegt und unter Rühren 36,8 g einer 20%igen Lösung von Natriumethylat in Ethanol zugetropft. Zu der Suspension werden bei 35°C 20,5 g 5-(4-Fluorphenyl)-pyridin-3-carbaldehyd gegeben und weitere 3 Stunden gerührt. Nach Zugabe von 4,2 g Natriumborhydrid wird weitere 4 Stunden gerührt und dann bei Raumtemperatur 62 ml Wasser zugetropft. Danach wird der pH-Wert der Reaktionsmischung mit 37%iger Salzsäure innerhalb von einer Stunde auf pH 4 eingestellt. Die Kristalle werden abfiltriert, mit Ethanol nachgewaschen und im Vakuum getrocknet. Man erhält 27.2 g (R)-(-)-2-[5-(4-Fluorphenyl)-3-pyridylmethylaminomethyl]-chroman Hydrochlorid (69 % Ausbeute).

### Beispiel 2:

20,27 g 2-Aminomethylchroman Hydrochlorid werden in 130 g Methanol vorgelegt und anschliessend mit 20,4 g einer 30%igen Natriumrnethylatlüsung in Methanol versetzt. Bei 35°C werden 20,43 g 5-(4-Fluorphenyl)-pyridin-3-carbaldehyd zu der weißen Suspension gegeben und 1,5 Stunden gerührt, bevor portionsweise 4,20 g Natriumborhydrid zugegeben werden. Nach 15 Stunden werden 56,4 ml Wasser zugegeben und der pH-Wert der Mischung mit 37%iger Salzsäure auf pH 2 eingestellt. Nach Abkühlen der Suspension auf 0°C werden die Kristalle abfiltriert, mit Methanol gewaschen und im Vakuum getrocknet. Man erhält 28.2 g 2-[5-(4-Fluorphenyl)-3-pyridylmethylaminomethyl]-chroman Hydrochlorid (64 % Ausbeute).

### Beispiel 3:

5,07 g (R)-2-Aminomethylchroman und 5,00 g 5-(4-Fluorphenyl)-pyridin-3-carbaldehyd werden in 38 ml THF gelöst und unter Rühren mit 6 g 5%igem Palladium auf Aktivkohle versetzt. Unter Rühren wird bei Raumtemperatur unter Normaldruck hydriert. Nach beendeter Wasserstoffaufnahme wird der Katalysator abfiltriert. Man erhält 8,66 g (R)-(-)-2-[5-(4-Fluorphenyl)-3-pyridylmethylaminomethyl]-chroman durch Abdestillieren des Lösemittels (68% Ausbeute).

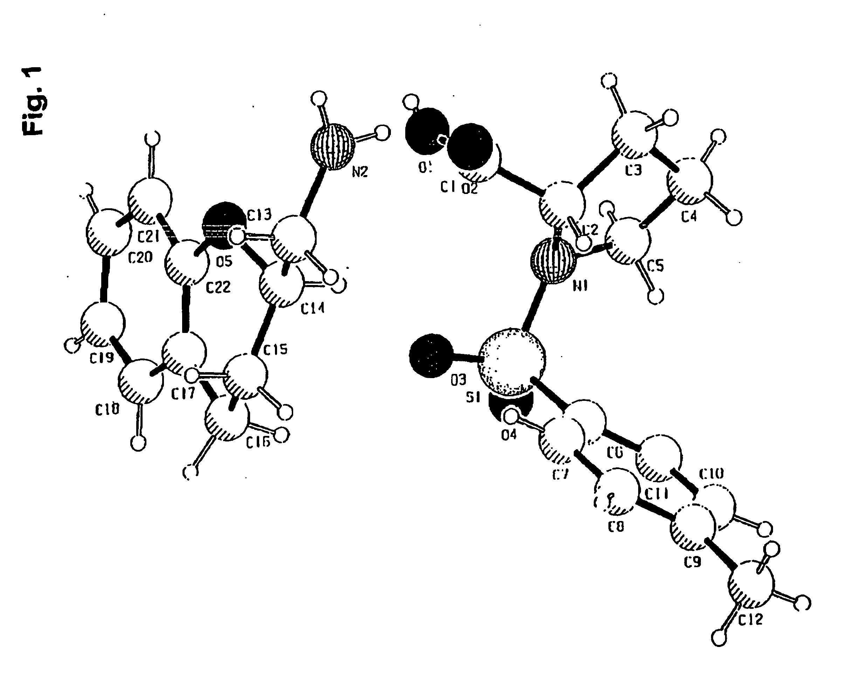

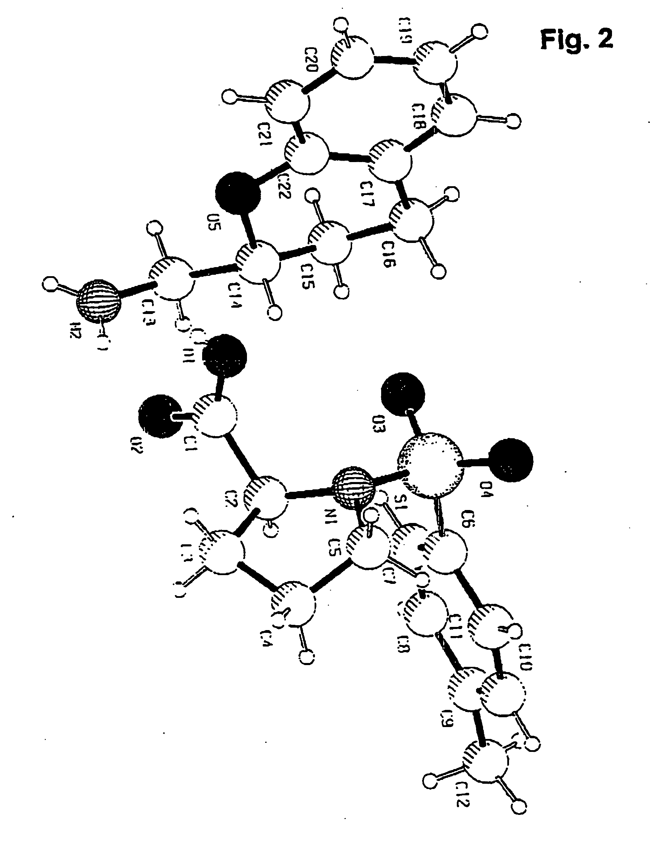

## Patentansprüche

1. Verfahren zur Herstellung von 2-[5-(4-Fluorphenyl)-3-pyridylmethylaminomethyl]-chroman der Formel I, 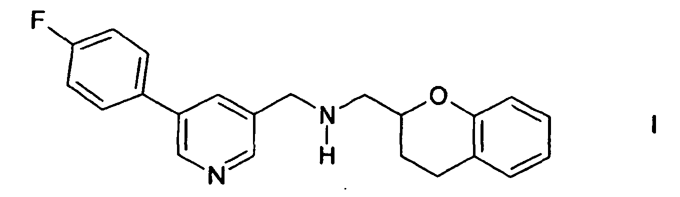 und dessen Salzen, **dadurch gekennzeichnet, dass**
(1) 5-(4-Fluorphenyl)-pyridin-3-carbaldehyd der Formel II 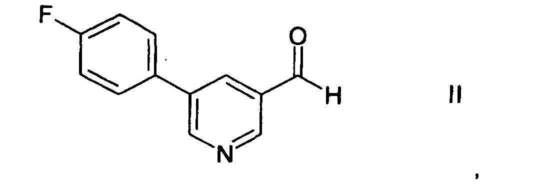 direkt mit 2-Aminomethyl-chroman oder dessen Salzen unter reduzierenden Bedingungen zu der Verbindung der Formel I umgesetzt wird und
(2) die erhaltene Verbindung der Formel I durch Behandeln mit einer Säure in eines ihrer Salze umgewandelt wird.

2. Verfahren nach Anspruch 1, **dadurch gekennzeichnet, dass** die reaktive Form des 2-Aminomethyl-chromans in situ aus einem Salz des Aminomethyl-chromans hergestellt wird.

3. Verfahren nach Anspruch 1 oder 2, **dadurch gekennzeichnet, dass** die Umsetzung in Gegenwart eines Borhydrids stattfindet.

4. Verfahren nach Anspruch 1 oder 2, **dadurch gekennzeichnet, dass** die Umsetzung in Gegenwart von Wasserstoff und einem Hydrierkatalysator stattfindet.

5. Verfahren nach einem oder mehreren der Ansprüche 1 bis 4, **dadurch gekennzeichnet, dass** (R)-Aminomethyl-chroman eingesetzt wird.

6. Verfahren nach einem oder mehreren der Ansprüche 1 bis 5, **dadurch gekennzeichnet, dass** (R)-Aminomethyl-chroman, hergestellt aus dem racemischen Aminomethyl-chroman durch Racematspaltung mit (S)-N-Tosylprolin eingesetzt wird.

7. Verfahren nach einem oder mehreren der Ansprüche 1 bis 6, **dadurch gekennzeichnet, dass** (R)-(-)-2-[5-(4-Fluorphenyl)-3-pyridylmethylaminomethyl]-chroman Hydrochlorid hergestellt wird.

8. Verfahren nach einem oder mehreren der Ansprüche 1 bis 6, **dadurch gekennzeichnet, dass** (R)-(-)-2-[5-(4-Fluorphenyl)-3-pyridylmethylaminomethyl]-chroman Dihydrochlorid hergestellt wird.

## Claims

1. Process for the preparation of 2-[5-(4-fluorophenyl)-3-pyridylmethylaminomethyl]chroman of the formula I 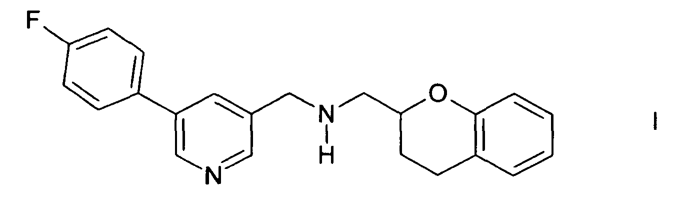 and salts thereof, **characterised in that**
(1) 5-(4-fluorophenyl)pyridine-3-carbaldehyde of the formula II 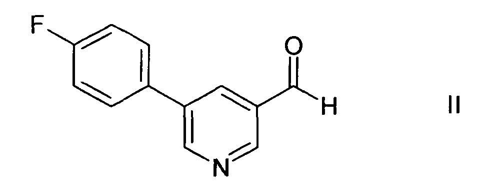 is reacted directly with 2-aminomethylchroman or salts thereof under reducing conditions to give the compound of the formula I, and
(2) the resultant compound of the formula I is converted into one of its salts by treatment with an acid.

2. Process according to Claim 1, **characterised in that** the reactive form of 2-aminomethylchroman is prepared in situ from a salt of aminomethylchroman.

3. Process according to Claim 1 or 2, **characterised in that** the reaction is carried out in the presence of a borohydride.

4. Process according to Claim 1 or 2, **characterised in that** the reaction is carried out in the presence of hydrogen and a hydrogenation catalyst.

5. Process according to one or more of Claims 1 to 4, **characterised in that** (R)-aminomethylchroman is employed.

6. Process according to one or more of Claims 1 to 5, **characterised in that** (R)-aminomethylchroman, prepared from racemic aminomethyl-chroman by racemate resolution using (S)-N-tosylproline, is employed.

7. Process according to one or more of Claims 1 to 6, **characterised in that** (R)-(-)-2-[5-(4-fluorophenyl)-3-pyridylmethylaminomethyl]chroman hydrochloride is prepared.

8. Process according to one or more of Claims 1 to 6, **characterised in that** (R)-(-)-2-[5-(4-fluorophenyl)-3-pyridylmethylaminomethyl]chroman dihydrochloride is prepared.

## Revendications

1. Procédé pour la préparation de 2-[5-(4-fluorophényl)-3-pyridylméthylaminométhyl]chroman de la formule I 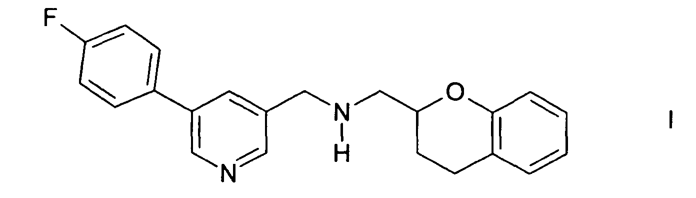 et de ses sels, **caractérisé en ce que** :
(1) 5-(4-fluorophényl)pyridine-3-carbaldéhyde de la formule II 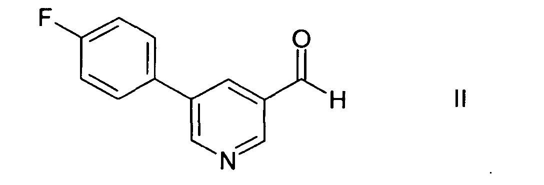 est amené à réagir directement avec 2-aminométhylchroman ou ses sels sous des conditions réductrices afin d'obtenir le composé de la formule 1 ; et
(2) le composé résultant de la formule I est converti selon l'un de ses sels par traitement avec un acide.

2. Procédé selon la revendication 1, **caractérisé en ce que** la forme réactive de 2-aminométhylchroman est préparée in situ à partir d'un sel d'aminométhylchroman.

3. Procédé selon la revendication 1 ou 2, **caractérisé en ce que** la réaction est mise en oeuvre en présence d'un borohydrure.

4. Procédé selon la revendication 1 ou 2, **caractérisé en ce que** la réaction est mise en oeuvre en présence d'hydrogène et d'un catalyseur d'hydrogénation.

5. Procédé selon une ou plusieurs des revendications 1 à 4, **caractérisé en ce que** du (R)-aminométhylchroman est utilisé.

6. Procédé selon une ou plusieurs des revendications 1 à 5, **caractérisé en ce que** du (R)-aminométhylchroman, préparé à partir d'aminométhylchroman racémique par resolution de racémate en utilisant (S)-N-tosylproline, est utilisé.

7. Procédé selon une ou plusieurs des revendications 1 à 6, **caractérisé en ce que** de l'hydrochlorure de (R)-(-)-2-[5-(4-fluorophényl)-3-pyridylméthylaminométhyl]chroman est préparé.

8. Procédé selon une ou plusieurs des revendications 1 à 6, **caractérisé en ce que** du dihydrochlorure de (R)-(-)-2-[5-(4-fluorophényl)-3-pyridylméthylaminométhyl]chroman est préparé.
